# EUROPEAN PATENT APPLICATION

(11) **EP 2 786 779 A1**
(43) Date of publication of application: **08.10.2014**
(21) Application number: 13162588.1
(22) Date of filing: 05.04.2013
(51) Int. Cl.: A61N 1/372, A61N 1/36, A61N 1/05

(54) **A system for planning and/or providing a therapy for neural applications**

(71) Applicant: Sapiens Steering Brain Stimulation B.V., 5656 AE Eindhoven (NL)
(72) Inventor: Aström, Mattias, 5509 KD Veldhoven (NL); Kleibeuker, Johan Gerard, 5268 HR Helvoirt (NL); Martens, Hubert Cécile Francois, 5611 ND Eindhoven (NL)
(74) Representative: Prinz & Partner

(57) **Abstract**

The present invention relates to a system (10) for planning and/or providing a therapy for neural applications, especially a neurostimulation and/or neurorecording applications, comprising at least one lead (300), the lead (300) having a plurality of electrodes (132) being capable to provide at least one stimulation field (F; F1; F2), further comprising at least one adjustment means (400), the adjustment means (400) being configured such that at least one characteristic parameter of the stimulation field (F; F1; F2) is directly and/or indirectly adjusted in order to establish at least one stimulation field (F; F1; F2) with at least one user defined maximal stimulation field characteristic at at least one user defined radial distance away from the lead (300).

## Description

The present invention relates to a system for planning and/or providing a therapy for neural applications, especially a neurostimulation and/or neurorecording applications.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin film leads are fixed on a stylet material to form a probe. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

In existing systems, the DBS lead has e.g. four 1.5 mm-wide cylindrical electrodes at the distal end spaced by 0.5 mm or 1.5 mm. The diameter of the lead is 1.27 mm and the metal used for the electrodes and the interconnect wires is an alloy of platinum and iridium. The coiled interconnect wires are insulated individually by fluoropolymer coating and protected in an 80A urethane tubing. With such electrode design, the current distribution emanates uniformly around the circumference of the electrode, which leads to stimulation of all areas surrounding the electrode.

The lack of fine spatial control over field distributions implies that stimulation easily spreads into adjacent structures inducing adverse side-effects in about 30% of the patients. To overcome this problem, systems with high density electrode arrays are being developed, hence providing the ability to steer the stimulation field to the appropriate target.

The clinical benefit of DBS is largely dependent on the spatial distribution of the stimulation field in relation to brain anatomy. To maximize therapeutic benefits while avoiding unwanted side-effects, precise control over the stimulation field is essential.

When programming neurostimulation devices the user typically define the active electrode contacts, the amplitude (current or electric potential) of one or more electrical sources that are applied to the active electrode contacts, as well as a few other electrical parameters that define the electrical pulses that are delivered to the patient. The electrical settings are then adjusted according to certain programming algorithms in order to reach an optimal therapeutic outcome.

Currently, the user has to manually and iteratively adjust the individual electrical settings in order to produce a field distribution with a specific field strength at a specific distance away from the stimulation lead.

It is therefore an object of the present invention, to improve a system for planning and/or providing a therapy for neural applications, especially a neurostimulation and/or neurorecording applications, in particular in that a field distribution of an stimulation field in connection with a therapy for neural applications with a specific field strength at a specific distance away from the stimulation lead may be set without iteratively adjust the individual electrical settings and that the adjusting may be done more intuitively.

The above object is solved according to the present invention by a system for planning and/or providing a therapy for neural applications according to claim 1. Accordingly, a system for planning and/or providing a therapy for neural applications, especially for neurostimulation and/or neurorecording applications, is provided comprising at least one lead, the lead having a plurality of electrodes being capable to provide at least one stimulation field, further comprising at least one adjustment means, the adjustment means being configured such that at least one characteristic parameter of the stimulation field is directly and/or indirectly adjusted in order to establish at least one stimulation field with at least one user defined maximal stimulation field characteristic at at least one user defined radial distance away from the lead.

By this, the advantage is achieved that a field distribution of an stimulation field in connection with a therapy for neural applications with a specific field strength at a specific distance away from the stimulation lead may be set without iteratively adjust the individual electrical settings and that the adjusting may be done more intuitively.

So, according to the invention it is now possible that during image-guided programming of neurostimulator devices, the stimulation lead and field may be visualized together with e.g. patient-specific anatomical images. With such information the advantage is achieved to let the user be in direct control of the distribution of the stimulation field, instead of the individual electrical settings.

By this invention a concept and system is provided that adjusts the stimulation amplitude in order to produce a stimulation field with a user defined maximal field strength, at a user defined radial distance away from the stimulation lead. The user defines the active electrode contacts of the lead where stimulation should be applied, as well as the desired field strength that should be distributed at a radial distance, while the system calculates and sets the electrical amplitude to produce such a field. This concept is from now on denoted as radial controlled programming.

The invention advantageously uses the fact that the distribution of the electric field and the stimulation amplitude(s) is substantially linear. When the stimulation amplitude(s) is doubled, then so is the electric field strength in the tissue. In order to perform radial controlled programming a finite element model of the stimulation lead and surrounding tissue may be used for simulations.

Especially, the lead may be a lead for neural applications, preferably a lead for a neurostimulation and/or neurorecording system. Such a neurostimulation and/or neurorecording system may be e.g. a DBS system.

The lead may e.g. comprise at least one thin film, whereby the thin film comprises a proximal end and a distal end, the lead further comprising a plurality of electrodes on the distal end of the thin film.

The thin film may include at least one electrically conductive layer, preferably made of a biocompatible material. The thin film may be assembled to the carrier and further processed to constitute the lead element. The thin film for a lead is preferably formed by a thin film product having a distal end, a cable with metal tracks and a proximal end. The distal end of the thin film may be forming a part of the distal end of the lead or substantially the distal end of the lead.

The distal end of the lead may be the end of the lead, which is in the implanted state of the lead the remote end of the lead with regard to the body surface area. In particular, in case of a lead for brain application, the distal end of the lead is the lower end of the lead, which is remote to the burr-hole of the skull, through which the lead is implanted.

There may be an Advanced Lead Can element, which may comprise electronic means to address the plurality of electrodes and at least one Advanced Lead Can connecting means. Further, the Advanced Lead Can element may be hermetically or substantially hermetically sealed and may comprise electronic means to address the plurality of electrodes on the distal end of the thin film, which is arranged at the distal end and next to the distal tip of the lead. The plurality of electrodes may comprise more than 5-10 electrodes, e.g. 16 or 32 electrodes or in preferred embodiments e.g. 64 electrodes or more. The electrodes may be arranged such that the electrodes are substantially evenly distributed arranged all over the distal end of the lead.

Additionally, it is possible that the adjustment means is configured such that the stimulation field is automatically directly and/or indirectly adjusted. By an automatic adjustment the adjustment process is improved and can be conducted by the user more easily.

Furthermore, it is possible that the at least one stimulation field characteristic is the activation of neurons caused by the at least one stimulation field and/or the field strength of the at least one stimulation field.

It is possible that the at least one characteristic parameter of the stimulation field is the stimulation amplitude and/or stimulation energy and/or the pulse-width.

By means of the stimulation amplitude and/or stimulation energy and/or the pulse-width it is advantageously possible to influence the activation of neurons caused by the at least one stimulation field. By means of the stimulation amplitude and/or stimulation energy it is advantageously possible to influence the field strength of the at least one stimulation field.

Furthermore, it is possible that the plurality of electrodes forms a complex geometrical array and/or that the plurality of electrodes is arranged circumferentially around at least one section of the lead, especially around a section next to the distal tip end of the lead.

By means of a complex array a stimulation field of any desired shape may be formed and may be adjusted according to the patient's needs. So, a suitable and accurate tailor-made stimulation field may be provided.

A complex array may be formed by a plurality of electrodes, which are arranged circumferentially around a section next to the distal tip end of the lead. The electrodes may be e.g. arranged non-planar and non-coaxial and likewise a leopard pattern and may form a regular array. Several electrodes may form at one level a ring around the lead and the next ring may be slightly displaced such that e.g. one electrode of the second ring is partially arranged within the gap between to electrodes of the first ring. So, there are rings of electrodes in radial direction of the lead and columns of electrodes in axial direction of the lead.

Furthermore, it possible that the adjustment means comprises at least one input means for inputting the at least one characteristic parameter of the stimulation field and that the adjustment means comprises at least one visualization means which is configured such that the lead and the at least one stimulation field can be visualized, wherein the input means and the visualization means are interconnected such that a geometrical interrelation of input and visualization is provided.

By this the advantage is achieved that the at least one characteristic parameter of the stimulation field can be intuitively input by the user, since due to the geometrical interrelation of input and visualization it is clear at which location of the stimulation field an amend of the at least one characteristic parameter of the stimulation field will cause an amendment of the stimulation field.

Advantageously, by means of the interconnection input means and visualization means and the geometrical interrelation of input and visualization, the effect of the change is immediately visualized in a "what-you-see-is-what-you-get-manner" and thus creating an intuitive input possibility combined with a fast and accurate adjustment of the stimulation field.

Moreover, it is possible that the visualization means is configured such that the at least one stimulation field is displayed around an axial top view of the lead.

So, beyond the constantly updated illustration of the stimulation field the user can be enabled to interpret the status of the stimulation field immediately.

It is further possible that the input means comprises one or more input points radially arranged around the visualization of the lead.

Moreover, it is possible that the geometrical interrelation of input and visualization is provided such that the visualization of the lead and the visualization of at least one stimulation field is arranged in the center of the input means, especially in the center of the circle of input points, wherein further especially the longitudinal axis of the lead being displayed in an axial top view is in the center of the circle of input points and the stimulation field is displayed on an isosurface level which is identical to the level defined by the input points.

So, the interconnection between input means and the visualization means is displayed and provides the possibility of an instant and intuitive adjustment of the stimulation field.

Further, also a simplified illustration of the stimulation field by an axial top view of the lead can be achieved, since the dimensions of the stimulation field are directly derivable. When the input means are embodied as one or more input points radially arranged around the visualization of the lead, an intuitive input and adjustment of the stimulation field is possible for the user.

Moreover, it is possible that the adjustment means comprises at least one touch screen, wherein the touch screen is configured such that the at least one input means and the at least one visualization means are provided by the touch screen.

It is further possible that the system comprises a simulation means, wherein the simulation means is capable to calculate and/or to simulate a stimulation field for a unit amplitude(s) applied to a specific set of active electrode contacts defined by the user.

The simulation means may comprise one or more controllers and/or processing means and/or the necessary calculation means and/or storing means and/or input means and/or output means to be capable that the simulation means is configured such that the shape of the at least one stimulation field can be modelled and/or simulated such that user input is transformed into the shape of the stimulation field.

Additionally it is possible that the system is configured such that the maximum electric field strength that should be distributed at a radial distance is defined by the user, especially via the adjustment means.

Moreover, it is possible that the system 10 is configured such that the maximum electric field strength is measured at the radial distance r in the finite element simulation, wherein especially the maximum electric field strength in the finite element simulation is measured by the simulation means and wherein especially the finite element simulation is simulated and provided by the simulation means.

Furthermore, it is possible that the simulation means is capable to calculate the ratio between the measured field strength and the desired field strength.

It is also possible that the simulation means is capable to multiply the unit amplitude(s) that was used during the simulation with the ratio between the measured field strength and the desired field strength and that the result is the amplitude required to produce the desired stimulation field.

Generally, it is also possible that the system is configured and arranged as system which is connectable to a lead of system for neural applications, especially for neurostimulation and/or neurorecording applications. Accordingly, the system for planning and/or providing a therapy for neural applications, especially a neurostimulation and/or neurorecording applications, is connectable to at least one lead, the lead having a plurality of electrodes being capable to provide at least one stimulation field, further comprising at least one adjustment means, the adjustment means being configured such that at least one characteristic parameter of the stimulation field is directly and/or indirectly adjusted in order to establish at least one stimulation field with at least one user defined maximal stimulation field characteristic at at least one user defined radial distance away from the lead.

If so, the system may comprise the system features as defined above and in claims 1 to 15.

Furthermore, the following method for planning and/or providing a therapy for neural applications, especially a neurostimulation and/or neurorecording applications is disclosed.

Accordingly, the method for planning and/or providing a therapy for neural applications, especially a neurostimulation and/or neurorecording applications comprises at least the step of simulating a stimulation field for a unit amplitude(s) applied to a specific set of active electrode contacts defined by the user.

Further, it is possible that the maximum electric field strength, E, that should be distributed at a radial distance, r, is defined by the user.

Moreover, it is possible that the maximum electric field strength is measured at the radial distance r in the finite element simulation.

Additionally, it is possible that the ratio between the measured field strength and the desired field strength is calculated.

Furthermore, the unit amplitude(s) that was used during the simulation is multiplied with this ratio. The result is the amplitude required to produce the desired field.

These steps may be combined and preferably all mentioned steps of the method for planning and/or providing a therapy for neural applications, especially a neurostimulation and/or neurorecording applications are carried out in the following order:
1. A stimulation field is simulated for a unit amplitude(s) applied to a specific set of active electrode contacts defined by the user.
2. The maximum electric field strength, E, that should be distributed at a radial distance, r, are both defined by the user.
3. The maximum electric field strength is measured at the radial distance r in the finite element simulation.
4. The ratio between the measured field strength and the desired field strength is calculated.
5. The unit amplitude(s) that was used during the simulation is multiplied with this ratio. The result is the amplitude required to produce the desired field.

In order to display the desired field without having to iterate the simulation of the field using the calculated amplitude, it is possible to display the field with an isosurface level corresponding to the measured field strength in the tissue. This can be done since the size and shape of that isosurface will be identical to the desired isosurface after recalculating the field with the calculated amplitude. Radial controlled stimulation can also be used to keep a constant but unspecified maximum field radius when the active electrode contact configuration is changed. In such case the radial distance, r, of the stimulation field is measured in the finite element simulation generated by the previous stimulation settings. This way the stimulation field radius is kept constant to the previous stimulation field settings.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe of a neurostimulation system for deep brain stimulation (DBS) and its components;
- Fig. 3:: a schematical drawing of a probe system according to the present invention;
- Fig. 4:: a first overview of a possible embodiment the system according to the invention; and
- Fig. 5:: a second overview of a possible embodiment the system according to the invention.

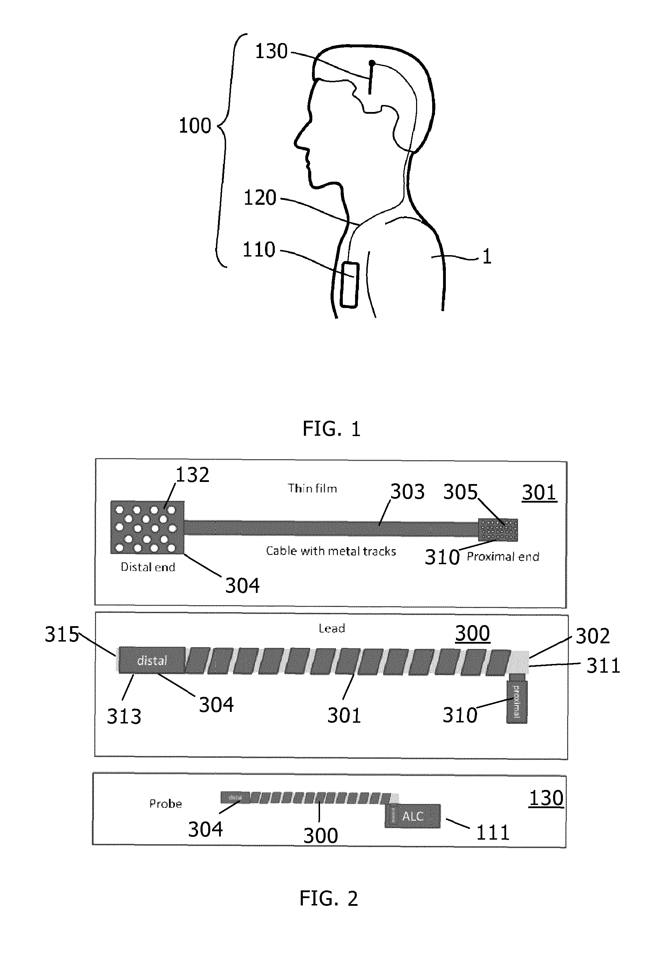

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Can element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the Advanced Lead Can element 111. The Advanced Lead Can element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

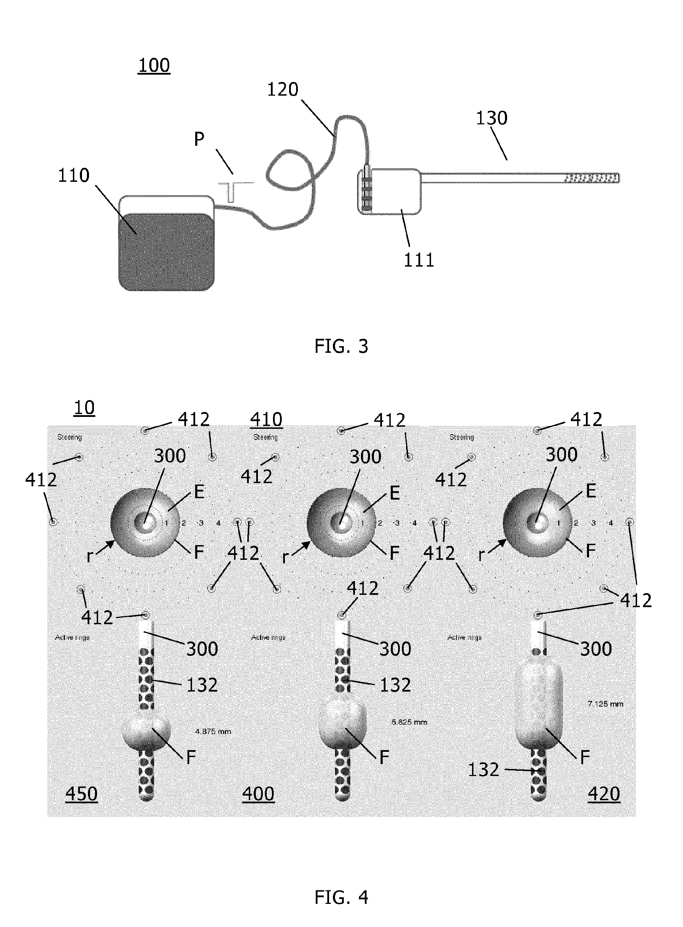

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the Advanced Lead Can 111. The controller 110 can be an implantable pulse generator (IPG) 110.

In Figure 4 a first overview of a possible embodiment of the system 10 according to the invention is illustrated.

The system 10 is a system for planning and/or providing a therapy for neural applications, here a system 10 for neurostimulation and/or neurorecording applications, namely a system 10 for planning and providing deep brain stimulation therapy.

The system 10 comprises a lead 300 having a plurality of electrodes 132. The electrodes 132 are capable to provide at least one stimulation field F; F1; F2.

The plurality of electrodes 132 forms a complex geometrical array and the plurality of electrodes 132 is arranged circumferentially around at least one section of the lead, here around a section next to the distal tip end of the lead 300.

The system 10 further comprises at least one adjustment means 400, the adjustment means 400 being configured such that at least one characteristic parameter of the stimulation field F; F1; F2 is directly and/or indirectly adjusted in order to establish at least one stimulation field F; F1; F2 with at least one user defined maximal stimulation field characteristic at at least one user defined radial distance away from the lead 300.

The adjustment means 400 is configured such that the stimulation field F; F1; F2 is automatically adjusted. The at least one stimulation field characteristic may be the activation of neurons caused by the at least one stimulation field F; F1; F2 and/or the field strength of the at least one stimulation field F; F1; F2.

The at least one characteristic parameter of the stimulation field F; F1; F2 may be the stimulation amplitude and/or stimulation energy and/or the pulse-width.

The adjustment means 400 includes at least one input means 410 for inputting the at least one characteristic parameter of the stimulation field F; F1; F2 and at least one visualization means 420 which is configured such that the lead and the at least one stimulation field F; F1; F2 can be visualized. The visualization means 420, based on a finite element model, is configured such that the at least one stimulation field F is displayed around an axial top view of the lead 300, which longitudinal axis represents the center of the circle of input points 412. The input means 410 are realized as one or more input points 412 radially arranged around the visualization of the lead 300, referred to as radially controlled stimulation.

In order to display the desired field without having to iterate the simulation of the field using the calculated amplitude, the field is displayed with an isosurface level corresponding to the measured field strength E in the tissue. Furthermore,

Figure 4 shows how the stimulation field F is affected when additional active electrode contacts 132 are activated (from left to right column).

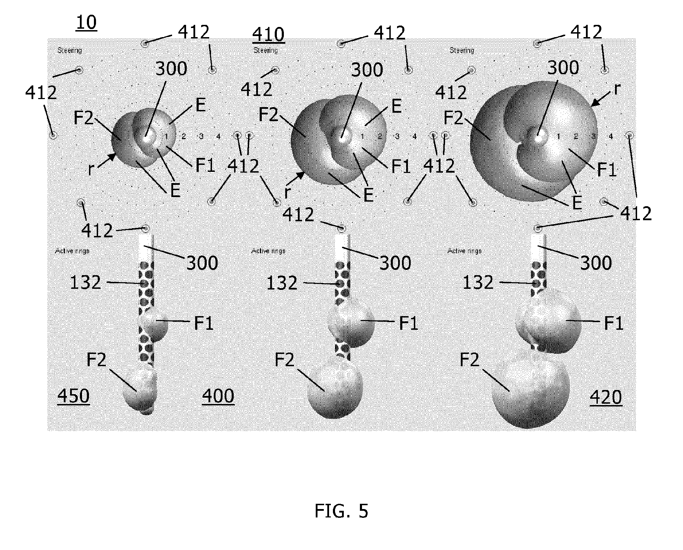

Figure 5 shows a second overview of a possible embodiment the system according to the invention as disclosed by Figure 4. Radial controlled programming is realized with several current sources to control an arbitrary stimulation field. When several sources are used the maximum stimulation field isosurface is aligned with the desired radius. The visualization is provided with an electric field isolevel.

As shown in Figure 4 and 5 there is a geometrical interrelation of input and visualization, i.e. the input means 410 with the input points 412 and the visualization of at least one stimulation field F; F1; F2 is geometrically interrelated.

The geometrical interrelation of input and visualization is provided such that the visualization of the lead and the visualization of at least one stimulation field is arranged in the center of the input means, here in the center of the circle of input points 412, wherein further especially the longitudinal axis of the lead 300 being displayed in an axial top view is in the center of the circle of input points 412 and the stimulation field is displayed on an isosurface level which is identical to the level defined by the input points 412.

The adjustment means 400 comprises at least one touch screen 450, wherein the touch screen 450 is configured such that the at least one input means 410 and the at least one visualization means 420 are provided by the touch screen 450. It is possible that the views shown in Figures 4 and 5 are displayed via the touch screen 450 and that substantially all user input may be input via the touch screen 450.

Also, the system 10 comprises a simulation means, wherein the simulation means is capable to calculate and/or to simulate a stimulation field for a unit amplitude(s) applied to a specific set of active electrode contacts defined by the user. The system 10 is configured such that the maximum electric field strength E that should be distributed at a radial distance r is defined by the user, especially via the adjustment means 400.

The system 10 is configured such that the maximum electric field strength is measured at the radial distance r in the finite element simulation, wherein especially the maximum electric field strength in the finite element simulation is measured by the simulation means and wherein especially the finite element simulation is simulated and provided by the simulation means. The simulation means is capable to calculate the ratio between the measured field strength and the desired field strength.

Furthermore, the simulation means is capable to multiply the unit amplitude(s) that was used during the simulation with the ratio between the measured field strength and the desired field strength and that the result is the amplitude required to produce the desired stimulation field.

In order to display the desired field without having to iterate the simulation of the field using the calculated amplitude, it is possible to display the field with an isosurface level corresponding to the measured field strength in the tissue. This can be done since the size and shape of that isosurface will be identical to the desired isosurface after recalculating the field with the calculated amplitude.

Radial controlled stimulation can also be used to keep a constant but unspecified maximum field radius when the active electrode contact configuration is changed. In such case the radial distance r of the stimulation field F, F1, F2 is measured in the finite element simulation generated by the previous stimulation settings. This way the stimulation field radius is kept constant to the previous stimulation field settings.

## Claims

1. A system (10) for planning and/or providing a therapy for neural applications, especially for neurostimulation and/or neurorecording applications, comprising at least one lead (300), the lead (300) having a plurality of electrodes (132) being capable to provide at least one stimulation field (F; F1; F2), further comprising at least one adjustment means (400), the adjustment means (400) being configured such that at least one characteristic parameter of the stimulation field (F; F1; F2) is directly and/or indirectly adjusted in order to establish at least one stimulation field (F; F1; F2) with at least one user defined maximal stimulation field characteristic at at least one user defined radial distance away from the lead (300).

2. The system (10) according to claim 1,
**characterized in that**
the adjustment means (400) is configured such that the stimulation field (F; F1; F2) is automatically directly and/or indirectly adjusted.

3. The system (10) according to claim 1 or 2,
**characterized in that**
the at least one stimulation field characteristic is the activation of neurons caused by the at least one stimulation field (F; F1; F2) and/or the field strength of the at least one stimulation field (F; F1; F2).

4. The system (10) according to one of the preceding claims,
**characterized in that**
the at least one characteristic parameter of the stimulation field (F; F1; F2) is the stimulation amplitude and/or stimulation energy and/or the pulse-width.

5. The system (10) according to one of the preceding claims,
**characterized in that**
the plurality of electrodes (132) forms a complex geometrical array and/or that the plurality of electrodes (132) is arranged circumferentially around at least one section of the lead, especially around a section next to the distal tip end of the lead (300).

6. The system (10) according to one of the preceding claims,
**characterized in that**
the adjustment means (400) comprises at least one input means (410) for inputting the at least one characteristic parameter of the stimulation field (F; F1; F2) and that the adjustment means (400) comprises at least one visualization means (420) which is configured such that the lead and the at least one stimulation field (F; F1; F2) can be visualized, wherein the input means and the visualization means are interconnected such that a geometrical interrelation of input and visualization is provided.

7. The system (10) according to claim 6,
**characterized in that**
the visualization means (420) is configured such that the at least one stimulation field is displayed around an axial top view of the lead (300).

8. The system (10) according to claim 6 or 7,
**characterized in that**
the input means (410) comprises one or more input points (412) radially arranged around the visualization of the lead (300).

9. The system (10) according to one of claims 6 to 8,
**characterized in that**
the geometrical interrelation of input and visualization is provided such that the visualization of the lead and the visualization of at least one stimulation field is arranged in the center of the input means, especially in the center of the circle of input points, wherein further especially the longitudinal axial of the lead (300) being displayed in an axial top view is in the center of the circle of input points (412) and the stimulation field is displayed on an isosurface level which is identical to the level defined by the input points (412).

10. The system (10) according to one of claims 6 to 9,
**characterized in that**
the adjustment means (400) comprises at least one touch screen (450), wherein the touch screen (450) is configured such that the at least one input means (410) and the at least one visualization means (420) are provided by the touch screen (450).

11. The system (10) according to one of the preceding claims,
**characterized in that**
the system (10) comprises a simulation means, wherein the simulation means is capable to calculate and/or to simulate a stimulation field for a unit amplitude(s) applied to a specific set of active electrode contacts defined by the user.

12. The system (10) according to one of the preceding claims,
**characterized in that**
the system (10) is configured such that the maximum electric field strength (E) that should be distributed at a radial distance (r) is defined by the user, especially via the adjustment means (400).

13. The system (10) according to one of the preceding claims,
**characterized in that**
the system (10) is configured such that the maximum electric field strength is measured at the radial distance (r) in the finite element simulation, wherein especially the maximum electric field strength in the finite element simulation is measured by the simulation means and wherein especially the finite element simulation is simulated and provided by the simulation means.

14. The system (10) according to one of claims 11 to 13,
**characterized in that**
the simulation means is capable to calculate the ratio between the measured field strength and the desired field strength.

15. The system (10) according to claim 14,
**characterized in that**
the simulation means is capable to multiply the unit amplitude(s) that was used during the simulation with the ratio between the measured field strength and the desired field strength and that the result is the amplitude required to produce the desired stimulation field.
